# EUROPÄISCHE PATENTANMELDUNG

(11) **EP 1 044 675 A2**
(43) Veröffentlichungstag der Anmeldung: **18.10.2000**
(21) Anmeldenummer: 00108275.9
(22) Anmeldetag: 14.04.2000
(51) Int. Cl.: A61K 7/46

(54) **Duftstoffapplikation**

(30) Priorität: 16.04.1999 DE 19917300
(71) Anmelder: drom fragrances international KG, 82065 Baierbrunn (DE); SWISS CAPS, SCA LOHNHERSTELLUNGS AG, CH-9533 Kirchberg (CH)
(72) Erfinder: Meller, Gerhard, 53879 Euskirchen (DE); Studer, Anton, 81479 München (DE); Maier, Hans J., Dr., 8638 Oberholz Ob Wald (CH)
(74) Vertreter: Patentanwälte Dr. Solf & Zapf

(57) **Zusammenfassung**

Es wird eine Duftstoffapplikation beschrieben, die eine Gelatinekapsel mit mindestens einem darin enthaltenem Duftstoff umfaßt, wobei die Duftstoffapplikation eine ausgezeichnete Lagerstabilität besitzt, und wobei durch Einstellung auf eine gewünschte Zerfallszeit der Gelatine-Kapseln eine zeitlich gesteuerte Freisetzung der Inhaltsstoffe der Gelatine-Kapseln ermöglicht wird.

## Beschreibung

Die Erfindung betrifft eine Duftstoffapplikation, mit der wenigstens ein Duftstoff auf gezielte und dosierte Weise freigesetzt werden kann.

Duftstoffe, bzw. Einzelriechstoffe und Riechstoffkompositionen werden schon seit Jahrhunderten für die unterschiedlichsten Beduftungen eingesetzt. Sie dienen beispielsweise zur Raumbeduftung, Körperbeduftung, zur Beduftung von Wasch-, Putz- und Reinigungsmitteln und Kosmetika. Dabei werden diese Produkte in Mehrdosenbehältnissen aus den unterschiedlichsten Materialien, insbesondere aus Glas- oder Kunststoff verpackt.

Es ist bekannt, Duftstoffe in beispielsweise aus Polyvinylpyrrolidon (PVP) bestehenden Mikrokapseln einzubringen und die Mikrokapseln auf ein geeignetes Trägermaterial aufzubringen, so daß beispielsweise Parfümproben geschaffen werden, bei denen durch Anwendung äußeren Drucks auf die Mikrokapseln diese zerbrechen und dabei den Duftstoff freisetzen.

Der Erfindung liegt die Aufgabe zugrunde eine lagerstabile Duftstoffapplikation zu schaffen, bei der einerseits der Duftstoff sicher gegen äußere Umwelteinflüsse abgeschirmt ist und andererseits, insbesondere in Wasser, leicht aus der Duftstoffapplikation freigesetzt werden kann.

Diese Aufgabe wird durch eine Duftstoffapplikation gelöst, die dadurch gekennzeichnet ist, daß sie eine Gelatinekapsel mit mindestens einem darin enthaltenen Duftstoff umfaßt.

Vorzugsweise besitzt der Duftstoff ein Molekulargewicht von mindestens 240, insbesondere mindestens 400 und ist ausgewählt aus der Duftstoffgruppe bestehend aus Alkoholen, Estern, insbesondere solche mit höherem Molekulargewicht, Ethern, Acetalen, Furanen, Makrocyclen, Schiffschen Basen, Lactonen, Pyranen, Alkenen und Thioverbindungen.

Beispiele für Duft-Alkohole sind Linalool, Phenylethylalkohol, Citronellol; Beispiele für Duft-Ester sind Methyldihydrojasmonat, Benzylacetat, ortho Butylcyclohexylacetat; Beispiele für Duft-Ether sind Methylphenylethylether, Isoamylphenylethylether, Diphenylether, Beispiele für Duft-Makrozyklen sind Cyclopentadecanoloide; Beispiele für Duft-Schiffsche Basen sind solche, die aus Methylanthranilat und Hydroxycitronellal, Methylanthranilat und Decanal und Methylanthranilat und Citronellal gebildet sind; Beispiele für Duft-Lactone sind Decalacton Gamma & Delta, Hexalacton Delta & Gamma; Beispiele für Duft-Thioverbindung sind Thiocineol, Thiogeraniol und als Beispiele für Duft-Pyrane können sämtliche Isomere von Rosenoxid sowie fast alle Rohstoffe mit einem Molekulargewicht von größer als 240 genannt werden.

Bei der Herstellung parfümhaltiger Gelatinekapseln waren grundsätzlich Komplikationen zu erwarten, da sowohl Parfüme wie auch Gelatine chemisch reaktive Zentren aufweisen, die störend in Wechselwirkung treten können. Ebenso können Probleme insofern auftreten, als Parfümbestandteile in die Gelatinematrix diffundieren und diese aufweichen, so daß die Stabilität von Weichgelatinekapseln nachteilig beeinflußt werden kann. Auch die Parfüme selbst sind störenden Einflüssen ausgesetzt, die durch Gelatineinhaltsstoffe herrühren und deren Eignung einschränken können, so daß ein beabsichtiger Verwendungszweck gefährdet ist, wobei es insbesondere nachteilig ist, daß die Reaktionen sehr langsam ablaufen und die Auswirkungen oft erst nach Monaten erkennbar werden.

Überraschenderweise wurde nun gefunden, daß die oben genannten, ausgewählten Duftstoffe keine negativen Auswirkungen auf die Gelatinekapsel besitzen, so daß sich lagerstabile Duftstoffapplikationen herstellen lassen, welche den Vorteil aufweisen, daß die Duftstoffe gegen äußere Umwelteinflüsse, wie Temperatur, Feuchtigkeit und dergleichen geschützt sind und andererseits die Gelatinekapseln bei der Lagerung nicht verspröden, aufweichen, schrumpfen oder zur Wasserunlöslichkeit neigen und somit die erfindungsgemäßen Duftstoffapplikationen ihre physikalischen Eigenschaften während der Lagerung nicht verändern.

Duftstoffe aus der Gruppe der Aldehyde, wie beispielsweise Hexanal, Hexylzimtaldehyd-alpha oder Duftstoffe aus der Gruppe der Ketone, wie beispielsweise Methylionon-gamma, Methylnonylketon und Ionon-alpha sowie der Terpine, wie beispielsweise Eukalyptol und Limonen führen dagegen zu Änderungen der physikalischen und chemischen Eigenschaften der Gelatinekapseln, so daß sich daraus keine lagerstabilen Duftstoffapplikationen herstellen lassen können.

Es wird angenommen, daß die Duftstoffe aus den oben angegebenen Duftstoffgruppen zu einer Stabilisierung der Gelatinestruktur führen, so daß die Gelatine ihren Feuchtigkeitsanteil auch während der Lagerung beibehält, und somit eine Versprödung oder ein Schrumpfen der Gelatinehülle vermieden wird.

Vorzugsweise kann die Gelatinekapsel der Duftstoffapplikation neben dem Duftstoff weiterhin ein Tensid enthalten, so daß die Duftstoffapplikation auch als Waschmittel, Allzweckreiniger, Spülmittel, Lufterfrischer und Körperpflegemittel eingesetzt werden kann oder der Duftstoff kann auch zusammen mit Pharmazeutika, Desinfektionsmitteln oder Konservierungsmitteln in die Gelatinekapsel eingefüllt und diese anschließend ihrem bestimmungsgemäßen Gebrauch durch Auflösen im Wasser zugeführt werden.

Der Fassungsinhalt der Gelatinekapsel beträgt vorzugsweise 0,1 bis 60 ml.

Bei der Anwendung der erfindungsgemäßen Duftstoffapplikation werden die Gelatinekapseln geöffnet und eine vordosierte Menge an Duftstoff und gegebenenfalls einer weiteren Komponente wird in ein gewünschtes Medium freigesetzt. Ein besonderer Vorteil bietet sich speziell dann, wenn die Gelatinekapseln komplett in eine vordosierte Menge Wasser gegeben und dort aufgelöst werden, um so eine gebrauchsfertige Lösung herzustellen. Ein Beispiel hierfür sind Gelatinekapseln, welche genau bemessene Mengen von parfümierten Waschmittelkonzentraten enthalten, die bei Eingabe in ein automatisches Waschsystem zusammen mit Wasser eine Waschlauge zur Reinigung von textilen Geweben ergeben.

Überraschenderweise wurde festgestellt, daß die Zerfallszeit der Gelatine durch Verwendung folgender, in Tabelle 1 genannter Duftstoffe und analoger Duftstoffe gesteuert werden kann:

**Tabelle 1**

| **Duftstoff** | **Zerfallszeit** |
|---|---|
| Methylnonylketon | 15 Min. |
| Damascon Alpha | 20 Min. |
| Orangen Terpene | 19 Min. |
| Terpinolen | 20 Min. |
| Eucalyptol | 20 Min. |
| Rose Oxid Inactive | 10 Min. |
| Florol 966.458 | 15 Min. |
| Isoamylphenylethylether | 18 Min. |
| Agrumex | 13 Min. |
| Verdylacetat | 17 Min. |
| Ald-C18 | 20 Min. |
| Tripial | 45 Min. |
| Eugenol | 60 Min. |
| MJ Gamma | 100 Min. |
| Benzyl Acetate | 110 Min. |

Die oben genannten Zerfallszeiten wurden für Duftstoffapplikationen gemessen, bei welchen die in Tabelle 1 genannten Duftstoffe in einer Menge von 1 Gew. -%, bezogen auf die Menge des eingesetzten Parfüms betrug.

Im allgemeinen beträgt die Menge des zusätzlich zum Parfüm-Anteil eingesetzten Duftstoffs 0,1 bis 20 Gew. -% um die Zerfallszeiten der Gelatine zu steuern.

Der Anteil des Parfüms in der Duftstoffapplikation beträgt im allgemeinen 0,5 bis 1,5 Gew.-%, bezogen auf die Gesamtmenge der Inhaltsstoffe der Duftstoffapplikation.

Mittels dieser Duftstoffe ist es möglich, bei einer Parfüm-Komposition die Weichgelatine auf eine gewünschte Zerfallszeit gemäß obigem Schema einzustellen und die Freisetzung der Inhaltsstoffe der Gelatine-Kapsel nach Bedarf zeitlich zu steuern.

Für den Fall, daß der Duftstoff in der Gelatinekapsel mit weiteren Komponenten wie Tensiden, Pharmazeutika, Desinfektionsmittel oder Konservierungsmittel nicht kompatibel ist, können auch Mischungen aus nur den Duftstoff enthaltenden Gelatinekapseln und den weiteren vorstehend genannten Komponenten hergestellt werden, so daß erst bei der bestimmungsgemäßen Anwendung der Mischung beim Auflösen in Wasser die Duftstoffe mit den weiteren Komponenten vermischt werden.

Für die Herstellung von Weichgelatinekapseln finden vorzugsweise bovine-, porcine- oder pescine-Gelatinen mit genügend hoher Gallertfähigkeit von 100 bis 250 Bloom Verwendung. Eine Basisrezeptur für Weichgelatinekapseln kann folgende Standardzusammensetzung aufweisen: 45% Gelatine (200 Bloom), 20% Glyzerin, 35% Wasser (gereinigt). Die Gelatinekapselherstellung erfolgt nach dem Rotary-Die-verfahren, wobei zunächst die flüssige, bei 60 °C gehaltene Gelatinemasse zu zwei Bandgieß-Vorrichtungen und durch präzis regulierbare Spalte geführt und durch Abkühlung auf Raumtemperatur zu zwei endlosen, elastischen Bändern mit definierter Dicke geformt wird. Diese Bänder werden von rechts und links zu einer zentralen Abfüll- und Verschließvorrichtung geführt. Die Bänder laufen tangential über zwei gegenseitig rotierende Formwalzen, welche Negativformate der Kapsel abbilden. In einem kontinuierlichen Vorgang werden unter mechanischem Druck der Formwalzen und unter Einwirkung von Wärme die doppellagigen Bänder zu taschenförmigen Gebilden verschweißt. In diese Taschen werden mittels Dosierpumpen vorgesehen Mengen an Füllgut eingepreßt. Die Kapseln werden dadurch gerade so weit aufgewölbt, wie ein Pumpenstoßfüllgut, d.h. Duftstoff oder Duftstoff zusammen mit einer weiteren Komponente wie Tensid, Pharamzeutikum, Desinfektionsmittel oder Konservierungsmittel in die Taschen drückt. Die Kapseln werden hierdurch vollkommene luftfrei gefüllt und verschlossen. Als Nachbehandlung werden die Kapseln im Umluftstrom schonend getrocknet und mit der Umgebungsfeuchte in ein Gleichgewicht gebracht.

## Patentansprüche

1. Duftstoffapplikation,
**dadurch gekennzeichnet,**
daß sie eine Gelatinekapsel mit mindestens einem darin enthaltenen Duftstoff umfaßt.

2. Duftstoffapplikation nach Anspruch 1,
**dadurch gekennzeichnet,**
daß der Duftstoff ein Molekulargewicht von mindestens 240, insbesondere mindestens 400 aufweist.

3. Duftstoffapplikation nach einem der Ansprüche 1 oder 2,
**dadurch gekennzeichnet,**
daß der Duftstoff ausgewählt ist aus der Duftstoffgruppe, bestehend aus Alkohlen, Estern, insbesondere solche mit höherem Molekulargewicht, Ethern, Acetalen, Furanen, Makrocyclen, Schiffschen Basen, Lactonen, Pyranen, Alkenen und Thioverbindungen.

4. Duftstoffapplikation nach einem oder mehreren der Ansprüche 1 bis 3,
**dadurch gekennzeichnet,**
daß weiterhin ein Tensid in der Gelatinekapsel enthalten ist.

5. Duftstoffapplikation nach einem oder mehreren der Ansprüche 1 bis 4,
**dadurch gekennzeichnet,**
daß die Gelatine, bovine-, porcine- oder pescine-Gelatine ist.

6. Duftstoffapplikation nach einem der Ansprüche 1 bis 5,
**dadurch gekennzeichnet,**
daß der Duftstoff und gegebenenfalls ein Tensid, Pharmazeutikum, Desinfektionsmittel oder Konservierungsmittel in der Gelatinekapsel in einer Menge von 0,1 bis 60 ml enthalten ist.

7. Duftstoffapplikation nach einem der Ansprüche 1 bis 6,
**dadurch gekennzeichnet,**
daß die Gelatinekapsel aus zwei verschweißten Gelatinekapselhälften besteht.

8. Duftstoffapplikation nach einem der Ansprüche 1 bis 7,
**dadurch gekennzeichnet,** daß
zur Steuerung einer gewünschten, unten angegebenen Zerfallszeit der Gelatine-Kapseln wenigstens ein weiterer Duftstoff, ausgewählt aus:
| | |
|---|---|
| Methylnonylketon | 15 Min. |
| Damascon Alpha | 20 Min. |
| Orangen Terpene | 19 Min. |
| Terpinolen | 20 Min. |
| Eucalyptol | 20 Min. |
| Rose Oxid Inactive | 10 Min. |
| Florol 966.458 | 15 Min. |
| Isoamylphenylethylether | 18 Min. |
| Agrumex | 13 Min. |
| Verdylacetat | 17 Min. |
| ald-C18 | 20 Min. |
| Tripial | 45 Min. |
| Eugenol | 60 Min. |
| MJ Gamma | 100 Min. |
| Benzyl Acetate | 110 Min. |
in den Gelatine-Kapseln enthalten ist.

9. Duftstoffapplikation nach Anspruch 8,
**dadurch gekennzeichnet,** daß
die Menge des weiteren Duftstoffs 0,1 bis 20 Gew.-%, bezogen auf die Menge des Parfüms in der Duftstoffapplikation, beträgt.

10. Duftstoffapplikation nach Anspruch 8 oder 9,
**dadurch gekennzeichnet,** daß
die Menge des weiteren Duftstoffs 1 Gew.-%, bezogen auf die Menge des eingesetzten Parfüms in der Duftstoffapplikation, beträgt.

11. Verwendung der Duftstoffapplikation nach einem der Ansprüche 1 bis 10, für Waschmittel, Allzweckreiniger, Spülmittel, Lufterfrischer, Körperpflegemittel, Pharmazeutika, Desinfektionsmittel und Konservierungsmittel.
